# EUROPEAN PATENT SPECIFICATION

(11) **EP 3 549 591 B1**
(45) Date of publication and mention of the grant of the patent: **03.11.2021**
(21) Application number: 19158828.4
(22) Date of filing: 18.08.2014
(51) Int. Cl.: A61K 35/28, A61P 1/02, A61P 29/00, C12N 5/0775

(54) **ADIPOSE-DERIVED MESENCHYMAL STEM CELLS FOR STOMATITIS TREATMENT**
ADIPOSE-MESENCHYMALE STAMMZELLEN ZUR STOMATITISBEHANDLUNG
CELLULES SOUCHES MÉSENCHYMATEUSES ADIPEUSES POUR LE TRAITEMENT DE LA STOMATITE

(30) Priority: 05.09.2013 US 201361874346 P
(43) Date of publication of application: 09.10.2019
(62) Divisional of application: 14841620.9
(73) Proprietor: The Regents of the University of California, Oakland, CA 94607 (US)
(72) Inventor: ARZI, Boaz, Davis California 95616 (US); BORJESSON, Dori L., Davis California 95618 (US); VERSTRAETE, Frank J.M., Davis California 95616 (US)
(74) Representative: Forresters IP LLP

(56) References cited:
- EP-A1- 1 421 957
- WO-A2-2012/142569
- US-A1- 2007 269 484
- US-A1- 2009 054 528
- US-A1- 2013 029 292
- US-A1- 2013 164 267
- JENNA N. WINER ET AL: "Therapeutic Management of Feline Chronic Gingivostomatitis: A Systematic Review of the Literature", FRONTIERS IN VETERINARY SCIENCE, vol. 3, 18 July 2016 (2016-07-18), XP055351418, DOI: 10.3389/fvets.2016.00054

## Description

### FIELD OF THE INVENTION

Provided are adipose-derived mesenchymal cells (AdMSCs) for use to prevent, mitigate, ameliorate and/or reverse stomatitis in a subject mammal in need therof. Methods for preparation of the MSCs are also disclosed (not claimed).

### BACKGROUND OF THE INVENTION

Feline chronic gingivostomatitis (FCGS) is an inflammatory syndrome affecting the mouth of cats (1-4). FCGS can be debilitating, difficult to treat, and may result in euthanasia of severely affected cats. The lesions are most commonly identified near the caudal aspect of the oral cavity at the palatoglossal folds, with extension along the buccal and gingival mucosa crossing the mucogingival junction (1 ;4). The clinical entity is thought to be a multifactorial condition where the host immune system is responding inappropriately to chronic oral antigenic stimulation including dental conditions, such as periodontal disease and dental resorption, and clinical or subclinical viral infections (4-9). Histological appearance of these lesions consists primarily of lymphocytes and plasma cells, with fewer neutrophils, macrophage-like cells and mast cells (1;2). Moreover, immunohistochemical profile of these lesions demonstrates mixed CD79a (B cells) and CD3 positive T cells. The level of MHC-II expression tends to correlate with the severity of inflammation (2).

Medical treatment of FCGS has been unrewarding with no specific treatment showing superiority (4). Tooth extraction, to reduce chronic oral antigenic stimulation, in the vicinity of alveolar mucositis and caudal stomatitis, as well as teeth suffering from periodontitis or resorptive lesions have shown the best results with 60% of the cats being clinically cured (4; 10). Cats with refractory disease require constant medical therapy such as antibiotics and anti-inflammatory drugs. Corticosteroids are often used to decrease oral inflammation and stimulate appetite. Recently, recombinant feline interferon omega has been suggested as a possible treatment for refractory FCGS cases (4).

Mesenchymal stem cells (MSCs) are adherent, adult-derived, fibroblast-like, multipotent stem cells (11). MSCs are defined by their surface protein expression and their ability to undergo tri-lineage differentiation (osteocyte, chondrocyte and adipocyte) (12-15). Although there is no single definitive MSC marker, a consensus is that MSCs should express CD44, CD 105 and CD90 and should not express CD45, CD34, CD80, CD86 or major histocompatibility class 2 (MHC-II) (13;15;16) MSCs reside in most organs and tissues investigated to date, including bone marrow, adipose, dermis, muscle, hair follicles, the periodontal ligament and the placenta (11; 17; 18). Adipose-derived MSCs (adMSCs) have been isolated and characterized from several veterinary species including cats (11-13;16;19-21).

MSCs have a profound regenerative ability attributed in part to their ability modulate both innate and adaptive immunity (17;22-26) Although the mechanism(s) of immunomodulation remain incompletely understood, MSCs inhibit T-cells proliferation, alter B-cell function, down-regulate MHC II and inhibit dendritic cell maturation and differentiation (14;22;23;25;26). Because of these functions, adMSCs are currently being used in phase II and III preclinical investigations for diseases including Crohn's disease and graft versus host disease with promising results (19;22;27-29). FCGS is characterized by lymphoplasmacytic inflammation and evidence of a dysregulated immune response (1;2).

### SUMMARY OF THE INVENTION

Provided is an effective amount of adipose-derived stem cells (AdMSCs) for the use in a method of preventing, reducing, mitigating, ameliorating and/or reversing stomatitis in a subject mammal in need thereof.

With respect to embodiments of the methods of treatment, in varying embodiments, the mammal is a feline. In some embodiments, the mammal is a human. In some embodiments, the mammal is a canine. In some embodiments, mammal is a non-human mammal, e.g., Felidae, Equidae, Bovidae, Cervidae, Suidae, Canidae, Rodentia, Lagomorpha, Camelidae, Ursidae, Procyonidae, Mustelidae, Elephantidae, or a non-human primate. In some embodiments, the stomatitis is persistent, chronic, recalcitrant and/or otherwise untreatable. In some embodiments, the stomatitis is acute. The stomatitis can be gingivostomatitis. In some embodiments, the MSCs are autologous to the mammal. In some embodiments, the MSCs are syngeneic to the mammal. In some embodiments, the MSCs are allogeneic to the mammal. In some embodiments, the MSCs are xenogeneic to the mammal. In varying embodiments, the MSCs are positive for the cell surface expression of one or more of CD44+, CD 105+ and CD90+, and negative for the cell surface expression of one or more of CD4-, CD34-, CD45-, CD80-, CD86- and MHC class II-. In varying embodiments, the MSCs have not been frozen. In some embodiments, the MSCs are fresh (i.e., not frozen) and viable. In varying embodiments, the population of MSCs administered is at least about 50% viable, e.g., at least about 55%, 60%, 65%, 70%, 75%, 80%, 85%, 90%, 95%, 98%, 99%, 100% viable. In some embodiments, the MSCs have been cultured in vitro for at least 1 passage, e.g., from about 2 to about 8 passages, e.g., 1, 2, 3, 4, 5, 6, 7 or 8 passages. In varying embodiments, the MSCs have been cultured in serum-free cell culture media. In varying embodiments, the MSCs have been cultured in cell culture media comprising serum proteins allogeneic to the mammal. In some embodiments, the MSCs are substantially free of serum proteins xenogeneic to the mammal. In some embodiments, the MSCs are substantially free of bovine serum proteins and/or equine serum proteins. In some embodiments, at least about 1 million MSCs/kg subject are administered, e.g., at least about 2 million, 2.5 million, 3 million, 3.5 million, 4 million, 5 million, 6 million, 7 million, 8 million, 9 million or 10 million MSCs/kg subject are administered or engrafted. In varying embodiments, about 1 million to about 10 million MSCs/kg subject, e.g., about 2 million to about 8 million MSCs/kg are administered or engrafted. In varying embodiments, at least about 5 million MSCs are administered, e.g., at least about 10 million, 15 million, 20 million, 25 million, 30 million, 35 million, 40 million, 45 million, 50 million, 55 million, 60 million, 65 million, 70 million, 75 million, 80 million, 85 million, 90 million, 95 million or 100 million MSCs are administered. In varying embodiments, about 5 million to about 100 million MSCs are administered, e.g., about 10 million to about 80 million MSCs are administered. In varying embodiments, the MSCs are administered at a rate of about 1 million to about 10 million cells per minute, e.g., at a rate of about 2 million to about 4 million cells per minute, e.g., at a rate of about 2.5 million to about 3.5 million cells per minute. In varying embodiments, the mammal is exhibiting symptoms of oral inflammation. In some embodiments, the MSCs are administered systemically. In some embodiments, the MSCs are administered intravenously. In varying embodiments, the MSCs are administered in multiple administrations, e.g., over 2, 3, 4, 5 or more administrations, as appropriate. In varying embodiments, the mammal has a CD4/CD8 ratio in blood that is less than about 1.0 prior to administration of the MSCs and a CD4/CD8 ratio in blood that is greater than about 1.3 after one or more administrations of the MSCs.

Described but not claimed are methods of preparing mesenchymal stem cells for administration to a feline. In some embodiments, the methods comprise culturing adipose-derived mesenchymal stem cells in cell culture medium comprising feline serum, wherein the cell culture medium is substantially free of or does not comprise serum proteins xenogeneic to the feline, e.g., is substantially free of or does not comprise bovine and/or equine serum proteins. In varying embodiments, the feline serum is allogeneic to the feline. In some embodiments, the cell culture medium is substantially free of or does not comprise bovine serum proteins or equine serum proteins.

Described but not claimed are methods of preparing mesenchymal stem cells for administration to a mammal. In some embodiments, the methods comprise culturing adipose-derived mesenchymal stem cells in cell culture medium comprising serum allogeneic or syngeneic to the mammal, wherein the cell culture medium is substantially free of or does not comprise serum proteins xenogeneic to the mammal. In varying embodiments, the serum is allogeneic to the mammal. In some embodiments, the cell culture medium is substantially free of or does not comprise bovine serum proteins or equine serum proteins. In varying embodiments, the MSCs have been cultured in serum-free cell culture media.

In certain embodiments, the methods comprise culturing adipose-derived mesenchymal stem cells in cell culture medium comprising serum xenogenic to the mammal. For example, in certain embodiments the methods comprise culturing adipose-derived mesenchymal stem cells from a feline in culture medium comprising fetal bovine serum.

In a further aspect, provided is a suspension of mesenchymal stem cells (MSCs) concentrated to at least about 1 × 10⁴/ ml, *e.g.,* at least about 5 x 10⁴/ ml, *e.g.,* at least about 1 × 105/ ml, *e.g.,* at least about 5 x 105/ ml, *e.g.,* at least about 1 × 106/ ml, *e.g.,* at least about 5 × 10⁶/ ml, *e.g.,* at least about 1 × 10⁷/ ml, *e.g.,* at least about 2 x 10⁷/ ml, wherein the MSCs are not frozen and are substantially free of serum proteins xenogeneic to the MSCs, *e.g.,* is substantially free of or does not comprise bovine and/or equine serum proteins. In some embodiments, the MSCs are feline. In certain embodiments, provided is a suspension of feline mesenchymal stem cells (MSCs) concentrated to at least about 1 × 10⁴/ ml, *e.g.,* at least about 5 x 10⁴/ ml, *e.g.,* at least about 1 × 10⁵/ ml, *e.g.,* at least about 5 x 10⁵/ ml, *e.g.,* at least about 1 × 10⁶/ ml, *e.g.,* at least about 5 × 10⁶/ ml, *e.g.,* at least about 1 × 10⁷/ ml, *e.g.,* at least about 2 x 10⁷/ ml, wherein the MSCs are not frozen, further comprising bovine and/or equine serum proteins. In some embodiments, the MSCs are adipose-derived. In some embodiments, the MSCs have not been frozen. Further provided are kits and containers (e.g., a vial, a tube, a bag, a pouch) comprising the suspension of MSCs (not claimed).

### DEFINITIONS

The terms "individual," "patient,", "subject" interchangeably refer to a mammal, for example, a human, a non-human primate, a domesticated mammal (e.g., a canine or a feline), an agricultural mammal (e.g., equine, bovine, ovine, porcine), or a laboratory mammal (e.g., rattus, murine, Lagomorpha, hamster).

As used herein, the terms "treating" and "treatment" refer to delaying the onset of, retarding or reversing the progress of, reducing the severity of, or alleviating or preventing either the disease or condition to which the term applies (*i.e.,* gingivostomatitis, stomatitis), or one or more symptoms of such disease or condition.

The term "mitigating" refers to reduction or elimination of one or more symptoms of that pathology or disease, and/or a reduction in the rate or delay of onset or severity of one or more symptoms of that pathology or disease, and/or the prevention of that pathology or disease.

The term "effective amount" or "therapeutically effective amount" refers to the amount of an active agent sufficient to induce a desired biological result (*e.g.,* prevention, delay, reduction or inhibition of one or more symptoms of an oral inflammation, *e.g.,* chronic oral inflammation, *e.g.,* gingivostomatitis, stomatitis, glossitis, mucositis). That result may be alleviation of the signs, symptoms, or causes of a disease, or any other desired alteration of a biological system. The term "therapeutically effective amount" is used herein to denote any amount of the formulation which causes a substantial improvement in a disease condition when applied to the affected areas repeatedly over a period of time. The amount will vary with the condition being treated, the stage of advancement of the condition, and the type and concentration of formulation applied. Appropriate amounts in any given instance will be readily apparent to those skilled in the art or capable of determination by routine experimentation.

A "therapeutic effect," as that term is used herein, encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

The terms "inhibiting," "reducing," "decreasing" with respect to oral inflammation refers to inhibiting one or more symptoms of the oral inflammation in a subject by a measurable amount using any method known in the art (e.g., visual inspection for amelioration of inflammatory lesions in the oral cavity, e.g., maxillary buccal mucosa, mandibular buccal mucosa, maxillary attached gingiva, mandibular attached gingiva, molar salivary gland, areas lateral to palatoglossal folds, oropharyngeal tissue, lingual and/or sublingual tissues), changes in blood markers (e.g., a CD4/CD8 ratio in blood that is greater than about 1.3), and behavioral changes in the subject (e.g., appetite, the ability to eat solid foods, grooming, sociability, activity levels, weight gain, exhibition of increased comfort). The one or more symptoms of oral inflammation are inhibited, reduced or decreased if the measurable parameter of the one or more symptoms is at least about 10%, 20%, 30%, 50%, 80%, or 100% reduced in comparison to the measurable parameter of the one or more symptoms prior to administration of the MSCs. In some embodiments, the measurable parameter of the one or more symptoms is inhibited, reduced or decreased by at least about 1-fold, 2-fold, 3-fold, 4-fold, or more in comparison to the measurable parameter of the one or more symptoms prior to administration of the MSCs.

The term "mesenchymal stem cells" refers to stem cells defined by their capacity to differentiate into bone, cartilage, and adipose tissue. With respect to cell surface markers, MSCs generally express CD44, CD90 and CD105, and do not express CD4, CD34, CD45, CD80, CD86 or MHC-II.

As used herein, "administering" refers to local and/or systemic administration, e.g., including enteral and parenteral administration. Routes of administration for the MSCs that find use in the present invention include, e.g., administration as a suppository, intravenous ("iv"), intraperitoneal ("ip"), intramuscular ("im"), intralesional, intranasal, or subcutaneous ("sc") administration. Administration may also be local to the target or damaged tissue, *e.g.,* to oral tissues including the oral mucosa and/or gingival tissue. Administration can be by any appropriate route such that the immunosuppressive agents secreted by the MSCs prevent, reduce or inhibit destruction or damage to the target tissue, *e.g.,* the oral mucosa, the gingiva and other tissues in the oral cavity. Parenteral administration includes, e.g., intravenous, intramuscular, intra-artcriolc, intraventricular, intradermal, subcutaneous, intraperitoneal, and intrarectal.

The terms "systemic administration" and "systemically administered" refer to a method of administering a compound or composition to a mammal so that the MSCs delivered to sites in the body, including the targeted site of pharmacological action, via the circulatory system. Systemic administration includes, e.g., intravenous, intra-arteriole, intraventricular intradermal, subcutaneous, intraperitoneal, and rectal administration.

The term "co-administer" and "co-administering" and variants thereof refer to the simultaneous presence of two or more active agents in the blood of an individual. The active agents that are co-administered can be concurrently or sequentially delivered.

The phrase "cause to be administered" refers to the actions taken by a medical professional (e.g., a physician), or a person controlling medical care of a subject, that control and/or permit the administration of the agent(s)/compound(s)/cell(s) at issue to the subject. Causing to be administered can involve diagnosis and/or determination of an appropriate therapeutic or prophylactic regimen, and/or prescribing particular agent(s)/compounds/cell(s) for a subject. Such prescribing can include, for example, drafting a prescription form, annotating a medical record, and the like.

The term "feline" refers to an animal that is a member of the family Felidae; including without limitation the subfamilies, Felinae, Pantherinae, and Acinonychinae; the genera Caracal, Catopuma, Felis, Herpailurus, Leopardus, Leptailurus, Lynx, Oncifelis, Oreailurus, Otocolobus, Prionailurus, Profelis, Puma, Neofelis, Panthera, Pardofelis, and Uncia; the species felis, lybica, jubatus, caracal, badia, bieti, chaus, margarita, nigripes, silvestris, gordonii, yaguarondi, pardalis, tigrinus, wiedi, serval, canadensis, lynx, pardinus, rufus, colocolo, geoffroyi, guigna, jacobita, manul, bengalensis, planiceps, rubiginosus, viverrinus, aurata, concolor, nebulosa, leo, onca, pardus, tigris, marmorata, and uncial.

### BRIEF DESCRIPTION OF THE DRAWINGS

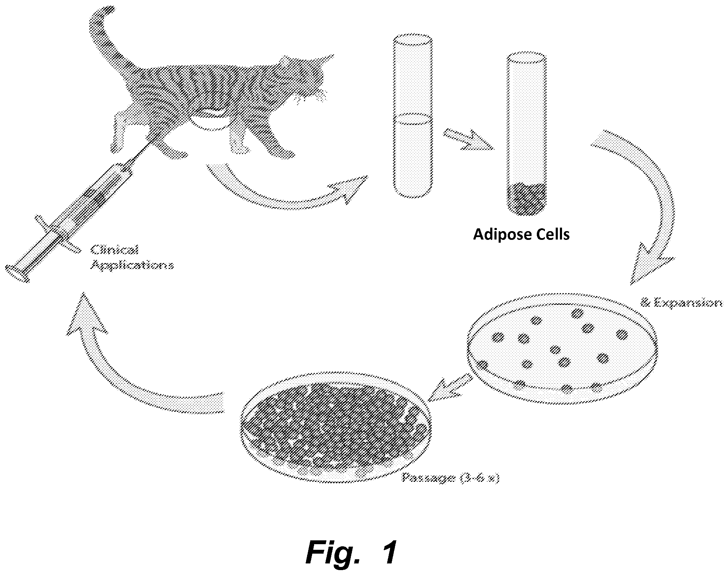

Figure 1 illustrates a schematic of the procedures for obtaining and administering MSCs to a subject mammal, e.g., a feline.

### DETAILED DESCRIPTION

### 1. Introduction

The present invention is based, in part, on the discovery of the efficacious use of mesenchymal stem cells (MSCs) for the prevention, mitigation and/or reversal of oral inflammation in a mammal, including the prevention, mitigation and/or reversal of oral inflammatory conditions selected from gingivostomatitis, stomatitis, glossitis and/or mucositis. The methods find use in the prevention, mitigation and/or reversal of feline chronic gingivostomatitis (FCGS). FCGS is a severe inflammatory oral disease of cats that is often refractory to treatment. The condition is marked by oral inflammation affecting the gingival and non-gingival mucosa, particularly the caudal oral mucosa lateral to the palatoglossal folds. Affected cats typically display clinical signs related to oral discomfort, including inappetence, anorexia and ptyalism. The etiopathogenesis of FCGS is poorly understood. Numerous factors such as breed predisposition, environmental stress, and a variety of infectious agents have been implicated in disease causation; however, the roles of these factors remain unresolved. Current treatment modalities include full or near-full mouth extractions, corticosteroid treatment, antibiotics and pain management. Recently, feline interferon treatment (available in Europe) has been proposed for non-responsive stomatitis. Extraction therapy provides the highest success rate (60%) whereas, 40% of the cases will either not respond to extractions or will need additional (often lifelong) medical management.

Mesenchymal stem cells (MSCs), including adipose-derived mesenchymal stem cells (adMSCs) and bone marrow-derived MSCs, provide regenerative and immunomodulatory capabilities combined with the ease of tissue attainment. Moreover, adMSCs have a significant advantage over other sources in generating larger number of cells. Herein, the therapeutic efficacy of systemic administration of MSCs to felines suffering from oral inflammation, in particular non-responsive FCGS cases, is demonstrated.

### 2. Subjects Who Can Benefit

The present methods can prevent, reduce, mitigate, ameliorate and/or reverse stomatitis in any mammal suffering from or at risk of suffering from gingivostomatitis or stomatitis. The mammal may or may not be exhibiting symptoms of stomatitis. In cases where the inflammation is in remission, the mammal may not be exhibiting symptoms. In other cases, the mammal may be suffering active inflammation, e.g. , that impedes the ability to eat and/or has resulted in tooth extractions.

In varying embodiments, the methods find use in preventing, reducing, mitigating, ameliorating and/or reversing stomatitis in a non-human mammal e.g., Felidae (e.g. , domesticated cat, cheetah, ocelot, lynx, bobcat, mountain lion, leopard, puma, lion, jaguar, tiger), Equidae (e.g., horse, ass, zebra), Bovidae (e.g., cattle, bison, sheep, goat, yak, impala, antelope, hartebeest, wildebeest, gnu, gazelle, water buffalo, duiker), Cervidae (e.g., deer, elk, moose, reindeer, pudu, bororo, brocket, guemal, muntjac), Suidae (e.g., pig, hog, boar), Canidae (domesticated dog, wolf, fox, coyote, jackal), Rodentia (e.g., mouse, rat, guinea pig, chinchilla, agouti, porcupine, beaver, gopher), Lagomorpha (e.g., rabbit, jackrabbit, hare, pika), Camelidae (e.g., camel, llama, alpaca, guanaco, vicugna), Ursidae (e.g., bear, panda), Procyonidae (e.g., raccoon, coati, olingo), Mustelidae (polecat, weasel, ferret, mink, fisher, badger, otter, wolverine, marten, sable, ermine), Elephantidae (e.g., elephant), rhinoceros, hippopotamus and non-human primates (e.g., chimpanzee, bonobo, macaque, ape).

In varying embodiments, the subject is a feline. The feline can be domesticated or non-domesticated. The domesticated feline can be a domestic short hair, domestic medium hair or domestic long hair. Certain breeds of domesticated cats are more susceptible to developing oral inflammation, e.g., a persistent, chronic, recalcitrant and/or otherwise untreatable oral inflammation, e.g., gingivostomatitis, stomatitis, glossitis and/or mucositis. Illustrative breeds of domesticated cats particularly susceptible to the development of oral inflammation, e.g., feline chronic gingivostomatitis, include without limitation Siamese, Abyssinians, Persians, Himalayans and Burmese. Illustrative breeds of domesticated cats that can benefit from the present methods include without limitation, Abyssinian, American Bobtail, American Bobtail Shorthair (SH), American Curl, American Curl Longhair (LH), American Shorthair, American Wirehair, Balinese, Bengal, Birman, Bombay, British Shorthair, British Longhair, Burmese, Chartreux, Colorpoint Shorthair, Cornish Rex, Cymric, Devon Rex, Egyptian Mau, European Burmese, Exotic Shorthair, Havana Brown, Himalayan, Japanese Bobtail, Japanese Bobtail Longhair, Korat, LaPerm, Maine Coon, Manx, Munchkin, Munchkin Longhair, Nebelung, Norwegian Forest Cat, Ocicat, Oriental Longhair, Oriental Shorthair, Persian, Peterbald, Pixiebob, Pixiebob Longhair, RagaMuffm, Ragdoll, Russian Blue, Scottish Fold, Scottish Fold Longhair, Selkirk Rex, Selkirk Rex Longhair, Siamese, Siberian, Singapura, Snowshoe, Somali, Sphynx, Thai, Tonkinese, Toyger, Turkish Angora, Turkish Van, Chausie, Savannah, Bambino, Donskey, Highlander, Highlander Shorthair, Kurilian Bobtail, Kurilian Bobtail Longhair, Minskin, Ojos Azules, Ojos Azules Longhair, Serengeti and Sokoke, and mixtures thereof. The felines can be pure breeds, partial breeds, mixed breeds or mongrels.

Patients amenable to treatment include individuals at risk of disease but not showing symptoms, as well as patients presently showing symptoms of oral inflammation.

In various disclosures for methods of treatment, the subject may already exhibit symptoms of disease or be diagnosed as having stomatitis, particularly a persistent, chronic, recalcitrant and/or otherwise untreatable stomatitis, i.e gingivostomatitis. For example, the subject may be exhibiting one or more symptoms, including inflammatory lesions in the oral cavity, e.g., maxillary buccal mucosa, mandibular buccal mucosa, maxillary attached gingiva, mandibular attached gingiva, molar salivary gland, areas lateral to palatoglossal folds, oropharyngeal tissue, lingual and/or sublingual tissues, changes in blood markers (e.g., a CD4/CD8 ratio in blood that is less than about 1.2), and/or behavioral changes in the subject (e.g., loss of appetite, the inability to eat solid foods, absent or reduced grooming, absent or reduced sociability, absent or reduced activity levels or exhibition of malaise, weight loss, exhibition of pain). In such cases, administration of MSCs can reverse or delay progression of and or reduce the severity of disease symptoms.

The effectiveness of treatment can be determined by comparing a baseline measure of a parameter of disease before administration of the MSCs is commenced to the same parameter one or more time points after MSCs have been administered. Illustrative parameters that can be measured include without limitation amelioration of inflammatory lesions in the oral cavity, e.g. , maxillary buccal mucosa, mandibular buccal mucosa, maxillary attached gingiva, mandibular attached gingiva, molar salivary gland, areas lateral to palatoglossal folds, oropharyngeal tissue, lingual and/or sublingual tissues, changes in blood markers (e.g., a CD4/CD8 ratio in blood that is greater than about 1.3), and behavioral changes in the subject (e.g., appetite, the ability to eat solid foods, grooming, sociability, activity levels, weight gain, exhibition of increased comfort), as indicators that the treatment is effective.

For the purposes of prophylaxis, the subject may be asymptomatic but have a risk or predisposition to developing an oral inflammation or oral inflammatory disorder. In such cases, administration of MSCs can prevent or delay onset of disease or progression of oral inflammation into later stages of disease, and/or reduce the severity of the disease once present.

### 3. Preparation and Administration of Mesenchymal Stem Cells

### a. Mesenchymal Stem Cells

The adipose tissue of an adult mammal is a repository of mesenchymal stem cells (MSCs). MSCs for use in the present methods are isolated from fat (i.e., adipose), using techniques known in the art. Illustrative techniques are described herein and reported in, e.g., Chung, et al., Res Vet Sci. (2012) 92(I):66-75; Toupadakis, et al., Am J Vet Res. (2010) 71(10): 1237-45. Depending on the stimulus and the culture conditions employed, these cells can form bone, cartilage, tendon/ligament, muscle, marrow, adipose, and other connective tissues.

The MSCs are derived from adipose tissue. Adipose-derived MSCs (AdMSCs) can be obtained from either autologous (self) and allogeneic (non-self) sources. The use of allogeneic MSCs in patients is possible due to their low immunogenicity. However, autologous AdMSCs are non-immunogenic and considered to be safe in people and animals. AdMSCs can be administered either systematically (e.g., intravenous or intraarterially) or locally (e.g., to any inflamed oral mucosa, e.g., into the gingiva, oral mucosal and/or periodontal tissues and/or other soft tissue of the oral cavity) in the treatment of disorders. Illustrative oral mucosal tissues that may be inflamed and ameliorated by local administration of MSCs include without limitation, e.g., maxillary buccal mucosa, mandibular buccal mucosa, maxillary attached gingiva, mandibular attached gingiva, molar salivary gland, areas lateral to palatoglossal folds, oropharyngeal tissue, lingual and/or sublingual tissues. MSCs can be generated more efficiently and rapidly from adipose tissue than from bone marrow. Fat- or adipose-derived MSCs are present in higher number and have a significantly higher proliferation rate then bone-marrow derived MSCs.

Generally, the MSCs useful for administration express on their cell surface CD44, CD90 and CD105 and do not express on their cell surface CD4, CD34, CD45, CD80, CD86 or MHC-II. In various embodiments, the MSCs are adipose-derived mesenchymal stem cells (AdMSC). AdMSCs can be characterized by the surface expression of CD5, CD44, CD90 (Thy-1) and CD 105; and by the non-expression of CD3, CD4, CD18, CD34, CD45, CD49d, CD80, CD86 and MHC class II. In other disclosures, the MSCs are derived from a non-adipose tissue, for example, bone marrow, liver, periodontal ligament, gingiva and/or dermal tissues. In some embodiments, the MSCs are non-hematopoietic stem cells derived from bone marrow (e.g., do not express CD34 or CD45).

As appropriate, the MSCs can be autologous (i.e., from the same subject), syngeneic (i.e., from a subject having an identical or closely similar genetic makeup); allogeneic (i.e., from a subject of the same species) or xenogeneic to the subject (i.e., from a subject of a different species).

In various embodiments, the MSCs may be altered to enhance the viability of engrafted or transplanted cells. For example, the MSCs can be engineered to overexpress or to constitutively express Akt. See, e.g., U.S. Patent Publication No. 2011/0091430.

### b. Preparation

Tissue comprising MSCs is obtained and processed. The tissue is adipose. The obtained MSCs can be autologous, syngeneic, allogeneic or xenogeneic to the subject mammal. The tissue is processed to isolate the MSCs such that they can be cultured in vitro. Solid tissues can be minced and digested with a proteinase {e.g., collagenase), as appropriate. In varying embodiments, the isolated MSCs can be cultured in vitro for at least 1 passage, e.g., from about 2 passages to about 8 passages, e.g. for 2, 3, 4, 5, 6, 7, 8 passages, as appropriate. At least 24 hours prior to injection into the subject, e.g., 24, 36 or 48 hours prior to injection, the cultured MSCs are washed and then cultured in media comprising serum of the same species of the subject mammal {e.g., autologous, syngeneic and/or allogeneic serum) and in the absence of serum xenogeneic to the subject mammal to wash out serum proteins allogeneic to the subject mammal in order to avoid or minimize the risk of a potential transfusion reaction. For example, the present MSCs can be substantially free of bovine and/or equine serum proteins prior to injection to a feline or canine subject. In some embodiments, the MSCs are cultured and/or resuspended in serum-free media. In certain embodiments, the isolated MSCs are cultured in media comprising xenogeneic serum {e.g., feline MSCs are cultured in medium comprising fetal bovine serum) and then the cells are washed prior to injection to remove serum proteins allogeneic to the subject mammal in order to avoid or minimize the risk of a potential transfusion reaction.

Prior to injection or engraftment into the subject mammal, the MSCs are rinsed and resuspended in a serum- free isotonic buffered solution {e.g., phosphate-buffered saline, 0.9% sodium chloride, lactated Ringer's solution (LRS), Hank's Balanced Salt Solution (HBSS), Earle's Balanced Salt Solution (EBSS), etc.).

Generally, the MSCs administered to the subject are fresh {i.e., not frozen) and viable. In varying embodiments, the MSCs have been cultured in vitro for one or more passages prior to injection, so the administered MSCs have never been frozen. The MSCs are evaluated for viability prior to administration. In varying embodiments, the population of administered MSCs are at least about 50% viable, e.g., at least about 55%, 60%, 65%, 70%, 75%, 80%, 85%, 90%, 95%, 98%, 99%, 100% viable.

### c. Administration

The MSCs can be administered by any appropriate route such that the anti-inflammatory and/or immunomodulatory factors secreted by the cells exert an anti-inflammatory and/or immune-inhibitory effect on the mediators of damage and/or destruction of the gingival and oral mucosal tissues and/or other soft tissues within the oral cavity. The anti-inflammatory and/or immune inhibitory effect can be local or systemic. In various embodiments, the MSCs are systemically administered, e.g., intravenously.

In some embodiments, the MSCs are administered locally, *e.g.,* into the gingiva, oral mucosal and/or periodontal tissues and/or other soft tissue of the oral cavity. As appropriate, the MSCs can be engrafted or transplanted into and/or around the gingiva, oral mucosal and/or periodontal tissues and/or other soft tissues of the oral cavity. When engrafted or transplanted into and/or in the vicinity of one or more tissues of the oral cavity, the MSCs are administered within or within sufficient proximity of the inflamed lesions in the oral cavity to exert an anti-inflammatory and/or immune-inhibitory effect on the mediators of damage and/or destruction of the gingival and oral mucosal tissue. For example, the MSCs are engrafted or transplanted into or within sufficient proximity to the tissues of the oral cavity such that any anti-inflammatory and/or immunosuppressive factors secreted by the MSCs prevent, reduce or inhibit damage and/or destruction to the tissues of the oral cavity, *e.g.,* the gingiva and/or the oral mucosal tissues.

As appropriate, injections of MSCs can be done after local anesthetics (e.g., lidocaine, bupivacaine) have been administered. It is also possible to inject the MSCs in conjunction with local anesthetics added to the cell suspension. Injections can also be made with the subject under general anesthesia with or without the use of local anesthetic agents (e.g., lidocaine).

In various embodiments, engraftment or transplantation of the MSCs can be facilitated using a matrix or caged depot. For example, the MSCs can be engrafted or transplanted in a "caged cell" delivery device wherein the cells are integrated into a biocompatible and/or biologically inert matrix (e.g. a hydrogel or other polymer or any device) that restricts cell movement while allowing the cells to remain viable. Synthetic extracellular matrix and other biocompatible vehicles for delivery, retention, growth, and differentiation of stem cells are known in the art and find use in the present methods. *See, e.g.,* Prestwich, J Control Release. (2011) 155(2):193-9; Perale, et al., Int J Artif Organs. (2011) 34(3):295-303; Suri, et al., Tissue Eng Part A. (2010) 16(5):1703-16; Khetan, et al., J Vis Exp. (2009) Oct 26;(32). pii: 1590; Salinas, et al., J Dent Res. (2009) 88(8):681-92; Schmidt, et al., J Biomed Mater Res A. (2008) 87(4):1113-22 and Xin, et al., Biomaterials (2007) 28:316―325.

As appropriate or desired, the engrafted or transplanted MSCs can be modified to facilitate retention of the MSCs at the region of interest or the region of delivery. In other embodiments, the region of interest for engraftment or transplantation of the cells is modified in order to facilitate retention of the MSCs at the region of interest or the region of delivery. In one embodiment, this can be accomplished by introducing stromal cell derived factor-1 (SDF-1) into the region of interest, e.g., using linkage chemistry or integrated biodegradable matrix (*e*.*g*., poly(D,L-lactide-co-glycolide (PLGA) beads) that would provide a tunable temporal presence of the desired ligand up to several weeks. MSCs bind to the immobilized SDF-1, thereby facilitating the retention of MSCs that are delivered to the region of interest for engraftment or transplantation. In other embodiments, integrating cyclic arginine-glycine-aspartic acid peptide into the region of interest can facilitate increased MSC binding and retention at the region of interest for engraftment or transplantation. *See*, *e.g.,* Ratliff, et al., Am J Pathol. (2010) 177(2):873-83.

In some embodiments, at least about 1 million MSCs/kg subject are administered or engrafted, e.g., at least about 2 million, 2.5 million, 3 million, 3.5 million, 4 million, 5 million, 6 million, 7 million, 8 million, 9 million or 10 million MSCs/kg subject are administered. In varying embodiments, about 1 million to about 10 million MSCs/kg subject, e.g., about 2 million to about 8 million MSCs/kg are administered or grafted.

In varying embodiments, at least about 5 million MSCs are administered or engrafted, e.g., at least about 10 million, 15 million, 20 million, 25 million, 30 million, 35 million, 40 million, 45 million, 50 million, 55 million, 60 million, 65 million, 70 million, 75 million, 80 million, 85 million, 90 million, 95 million or 100 million MSCs are administered or engrafted. In varying embodiments, about 5 million to about 100 million MSCs are administered or engrafted, e.g., about 10 million to about 80 million MSCs are administered or engrafted.

In varying embodiments, the MSCs are administered, e.g., intravenously, at a rate of about 1 million to about 10 million cells per minute, e.g., at a rate of about 2 million to about 4 million cells per minute, e.g., at a rate of about 2.5 million to about 3.5 million cells per minute.

A regime of treatment or prevention may involve one or multiple injections. For example, MSCs may be administered to the subject 1, 2, 3, 4, 5, 6, 7, 8, 9, 10, or more times, as appropriate. Subsequent administrations of MSCs may be administered systemically or locally. If administered locally, multiple injections of MSCs may be administered to the same or different locations. Multiple injections of MSCs can be administered daily, weekly, bi-weekly, monthly, bi-monthly, every 3, 4, 5, or 6 months, or annually, or more or less often, as needed by the subject. The frequency of administration of the MSCs can change over a course of treatment, e.g., depending on how well the engrafted or transplanted MSCs establish themselves at the site of administration and the responsiveness of the subject. The MSCs may be administered multiple times over a regime course of several weeks, several months, several years, or for the remainder of the life of the subject, as needed or appropriate.

The total amount of cells that are envisioned for use depend upon the desired effect, patient state, and the like, and may be determined by one skilled within the art. Dosages for any one patient depends upon many factors, including the patient's species, size, body surface area, age, the particular MSCs to be administered, sex, scheduling and route of administration, general health, and other drugs being administered concurrently.

### 4. Monitoring Efficacy

Clinical efficacy can be monitored using any method known in the art. Measurable parameters to monitor efficacy include, but are not limited to, visual inspection of amelioration of inflammatory lesions in the oral cavity, e.g., maxillary buccal mucosa, mandibular buccal mucosa, maxillary attached gingiva, mandibular attached gingiva, molar salivary gland, areas lateral to palatoglossal folds, oropharyngeal tissue, lingual and/or sublingual tissues), changes in blood markers (e.g., a CD4/CD8 ratio in blood that is greater than about 1.3), and behavioral changes in the subject (e.g., appetite, the ability to eat solid foods, grooming, sociability, activity levels, weight gain, exhibition of increased comfort). These parameters can be measured using any methods known in the art. The different parameters can be assigned a score, and the scores can be combined to provide a Stomatitis Disease Activity Index for the subject. Input can be provided by the client/caretaker/owner of the mammal and/or by self-reporting (in cases where the subject is a human) and/or by a trained clinician.

Observation of the stabilization, improvement and/or reversal of one or more symptoms or parameters by a measurable amount indicates that the treatment or prevention regime is efficacious. Observation of the progression, increase or exacerbation of one or more symptoms indicates that the treatment or prevention regime is not efficacious. For example, observation of the amelioration of one or more inflammatory lesions in the oral cavity, e.g., maxillary buccal mucosa, mandibular buccal mucosa, maxillary attached gingiva, mandibular attached gingiva, molar salivary gland, areas lateral to palatoglossal folds, oropharyngeal tissue, lingual and/or sublingual tissues), changes in blood markers (*e.g*., a CD4/CD8 ratio in blood that is greater than about 1.3), and behavioral changes in the subject (e.g., increased appetite, the ability to eat solid foods, improved/increased grooming, improved/increased sociability, improved/increased activity levels, weight gain and/or stabilization, exhibition of increased comfort) after one or more administrations of MSCs indicates that the treatment or prevention regime is efficacious. Likewise, observation of increased numbers or severity of inflammatory lesions in the oral cavity, e.g., maxillary buccal mucosa, mandibular buccal mucosa, maxillary attached gingiva, mandibular attached gingiva, molar salivary gland, areas lateral to palatoglossal folds, oropharyngeal tissue, lingual and/or sublingual tissues), changes in blood markers (e.g., a CD4/CD8 ratio in blood that is less than about 1.2), and behavioral changes in the subject (e.g., decreased appetite, the inability to eat solid foods, reduced or absent grooming, reduced or absent sociability, reduced or absent activity levels, weight loss, exhibition of pain and/or malaise), after one or more administrations of MSCs indicates that the treatment or prevention regime was not efficacious.

In certain embodiments, the monitoring methods can entail determining a baseline value of a measurable biomarker or disease parameter in a subject before administering a dosage of the one or more active agents described herein, and comparing this with a value for the same measurable biomarker or parameter after a course of treatment.

In other methods, a control value (i.e., a mean and standard deviation) of the measurable biomarker or parameter is determined for a control population. In certain embodiments, the individuals in the control population have not received prior treatment and do not have an inflammatory disorder of the oral cavity (e.g., chronic oral inflammation, e.g., gingivostomatitis, stomatitis, glossitis and/or mucositis), nor are at risk of developing an inflammatory disorder of the oral cavity (e.g., chronic oral inflammation, e.g., gingivostomatitis). In such cases, if the value of the measurable biomarker or clinical parameter approaches the control value, then treatment is considered efficacious. In other embodiments, the individuals in the control population have not received prior treatment and have been diagnosed with an inflammatory disorder of the oral cavity i.e. gingivostomatitis or stomatitis. In such cases, if the value of the measurable biomarker or clinical parameter approaches the control value, then treatment is considered inefficacious.

In other methods, a subject who is not presently receiving treatment but has undergone a previous course of treatment is monitored for one or more of the biomarkers or clinical parameters to determine whether a resumption of treatment is required. The measured value of one or more of the biomarkers or clinical parameters in the subject can be compared with a value previously achieved in the subject after a previous course of treatment. Alternatively, the value measured in the subject can be compared with a control value (mean plus standard deviation) determined in population of subjects after undergoing a course of treatment. Alternatively, the measured value in the subject can be compared with a control value in populations of prophylactically treated subjects who remain free of symptoms of disease, or populations of therapeutically treated subjects who show amelioration of disease characteristics. In such cases, if the value of the measurable biomarker or clinical parameter approaches the control value, then treatment is considered efficacious and need not be resumed. In all of these cases, a significant difference relative to the control level (i.e., more than a standard deviation) is an indicator that treatment should be resumed in the subject.

### 5. Kits (not claimed)

Provided are kits comprising mesenchymal stem cells (MSCs). In varying embodiments, the MSCs are fresh (i.e., not frozen) and viable. In varying embodiments, the MSCs have never been frozen. In varying embodiments, the kits comprise a population of MSCs that is at least about 50% viable, e.g., at least about 55%, 60%, 65%, 70%, 75%, 80%, 85%, 90%, 95%, 98%, 99%, 100% viable. In some embodiments, the MSCs are substantially free of serum proteins xenogeneic to the MSCs, e.g., free of bovine serum proteins and/or equine serum proteins. In varying embodiments, the MSCs are feline. In varying embodiments, the MSCs are adipose-derived or bone marrow-derived. In some embodiments, the MSCs are provided in an appropriate container, e.g., a vial, a tube, a pouch, a bag.

### EXAMPLES

The following examples are offered to illustrate, but not to limit the claimed invention.

### Example 1

### Assessment of the Efficacy of Autologous AdMSC in Cats with Non-Responsive Feline Chronic Gingivostomatitis (FCGS)

*Inclusion criteria*: Cats affected by FCGS that have undergone full standard of care therapy (full or near-full mouth extractions) but are non-responsive as indicated by ongoing oral inflammation and discomfort with ongoing medical therapy (antibiotics, pain medications and/or corticosteroids). Cats are otherwise healthy (e.g., normal physical examination, CBC biochemistry profile and urinalysis). These cats may have been treated with corticosteroids, antibiotics and/or pain medication however cats participating in the "treatment group" are free of corticosteroids and antibiotic treatments for at least 7 days prior to AdMSC therapy. Treatment of pain with opioids continues as needed. In addition, owners must commit to recheck visits.

*Exclusion criteria*: Cats that are affected by FCGS but have not received the current accepted standard of care (periodontal treatment and extractions). Cats that have concurrent disease (e.g., diabetes, renal disorders) or cats that have significant alterations in their minimum data base (CBC, serum biochemistry and urinalysis) with the exception of expected alterations in inflammatory parameters (increases in protein or globulin concentration or alterations in leukocyte numbers).

*Patient enrollment and baseline information*: All clients with cats that meet the inclusion criteria with non-responsive FCGS are offered enrollment in the study. If they consent, their cats are enrolled. Enrolled cats are randomly assigned to either the control group (continued receipt of the standard of care treatments, antibiotics and/or corticosteroids) or the treatment group. A minimum database (CBC, Chemistry profile and urinalysis) is obtained. Both control and treatment cats have fat collected and a thorough oral examination performed under general anesthesia. The extent of the disease is determined and the absence of concurrent oral diseases (e.g., neoplasia) is confirmed. The ventral abdomen just caudal to the umbilicus is surgically prepared. A 2-3 cm incision is made and 5-10 grams of subcutaneous adipose tissue are collected in a 50-ml tube containing 15ml PBS solution. Fat is immediately processed.

*Serum collection.* Cats from the William R. Prichard Veterinary Medical Teaching Hospital's pathogen free cat colony are used as serum donors. Animals are sedated with an intramuscular dose of 5mg/kg ketamine and 0.2mg/kg diazepam. This is followed by 10-20mg ketamine IV as calculated by the donor's weight. No more than 60 mL of blood is collected from the jugular vein using a vacutainer needle holder (BD, Franklin Lakes, NJ), a 20-gauge needle and serum separator tubes (Corvac Mansfield, MA). After collection, the blood is allowed to sit for 20 minutes and then it is spun at 3000 RPM for 5 minutes. Serum supernatant in the tubes is collected sterilely in a biosafety cabinet.

*Fat processing and culture.* Feline adipose tissue is collected and placed in sterile saline for processing. Briefly, the adipose tissue is minced with a razor blade and digested with 1mg/ml collagenase type I (Worthington, Lakewood, NJ)/bovine serum albumin (Fisher, Fair Lawn NJ) solution (0.1% collagenase type 1/1% BSA in DPBS) for 4 hours at 37°C in a water bath with shaking every 15 minutes. The sample is centrifuged (800 RPM for 10 minutes), the lipid layer is removed and discarded and the stromal vascular fraction (cellular fraction) is collected. The cellular fraction is washed once with saline and spun at 1200 RPM for 10 minutes to pellet the cells. The cells are plated in media containing low-glucose DMEM (Corning, Manassas, VA) supplemented with 10% FBS (HyClone, S. Logan, UT) and 1% penicillin streptomycin (Gibco, Grand Island, NY). Cells are cultured in an incubator with standard culture conditions (5% CO2, 37°C). Cells are passaged at 70-80% confluence, changing media every 48 hours. The cells are harvested with trypsin and passaged until adequate cell numbers arc obtained for injection (passage 2-5 cells are used). One dose is prepared for each treatment cat, the remaining MSC for both treatment and control cats are cryopreserved. Twenty four hours prior to treatment, cells are washed using Dulbecco's' Phosphate Buffered Saline (DPBS, Gibco, Grand Island, NY). The media containing 10% FBS is then replaced with media containing low-glucose DMEM supplemented with 10% allogeneic feline serum (collected and processed as described above) with 1% streptomycin. In certain embodiments, cells are cultured for 24 hours in media plus allogeneic feline serum to wash out bovine proteins (that can be adsorbed during extended cell culture in FBS) and therefore minimize the risk of a transfusion reaction.

*MSC characterization:* MSCs are characterized by surface marker expression using flow cytometry and a panel of feline-specific and cross-reactive antibodies specific for surface determinants expressed by MSC from other species. Specifically, feline MSC is analyzed for positive surface expression of CD44, CD105 and CD90 and negative surface expression of CD4 and feline MHC class 2.

*In vitro* differentiation assays are conducted to confirm the multipotency of feline MSCs. The cells' ability to differentiate into 3 cell lineages that are characteristic of MSC (fat, bone, cartilage) is assessed. These assays are performed by incubating confluent MSC with medium supplemented with factors to stimulate differentiation. At the end of the differentiation period, cells are fixed and stained with Oil Red O for presence of lipids, Masson trichrome for cartilage matrix, and alkaline phosphatase for the presence of calcium. MSCs cultured in MSC media alone under the same conditions are used as differentiation control.

*Patient treatment, assessment and follow up*: All cats are presented at approximately 2 weeks following fat harvest. For treatment cats, autologous MSCs (20 million cells per dose, suspended in LRS) are administered slowly IV. Control cats receive a single IV injection of LRS. Scores are reached by consensus by at least two veterinarians.

*The "stomatitis disease activity index form*" is completed for each patient prior to treatment and at each recheck visit. This index is a semi-quantitative scoring sheet that is used to grade the severity of disease, based on oral inflammatory parameters, and quality of life, based on owner-reported parameters. The cats are rechecked at two-weeks following initiation of therapy and then every 4 weeks for a total follow-up period of six months. At each visit, clients are asked to fill out the client section of the "stomatitis index form." At each visit, the veterinarian fills out the doctor section of the "stomatitis index form". A minimum database (CBC, biochemical profile and UA) is performed at the initial visit, 2 weeks after MSC administration and at the conclusion of the study (6 months after injection).

The criteria for the stomatitis disease activity index are provided below. Input is provided by client/owner and clinician.
**Appetite:** 3 = eats only pureed food, or only when hand fed 2 = eats wet food on his/her own; cannot eat dry food 1 = eating wet and dry food, but less than normal amount 0 = eating normally
**Activity level:** 3 = no interest in people or other pets, spends most of time sleeping 2 = low activity level, but will play occasionally when engaged by people or other pets 1 = plays spontaneously, but not frequently 0 = normal activity level (playful and active)
**Grooming behavior:** 3 = will not groom 2 = grooms occasionally but not at 'pre-illness' level 1 = grooming excessively 0 = grooming normally
**Perceived comfort:** On a scale of 0-3, with 0 being most comfortable and 3 being most painful, ranking of cat's present comfort level.
**Weight:** 0 = gain >0.5kg 1 = gain >0.25kg but <0.5kg 2 = <0.25kg gain 3 = weight loss (compared with most recent visit)
**Inflammation of oral cavity sites:** 0 = none 1 = mild 2 = moderate 3 = severe

**TABLE 1**

| **STOMATITIS DISEASE ACTIVITY INDEX** | **0** | **1** | **2** | **3** |
|---|---|---|---|---|
| Owner evaluation | | | | |
| Weight | | | | |
| Maxillary buccal mucosal inflammation | | | | |
| Mandibular buccal mucosal inflammation | | | | |
| Maxillary attached gingival inflammation | | | | |
| Mandibular attached gingival inflammation | | | | |
| Molar salivary gland inflammation | | | | |
| Inflammation of areas lateral to palatoglossal folds | | | | |
| Oropharyngeal inflammation | | | | |
| Lingual and/or sublingual inflammation | | | | |
| **TOTAL SCORE (maximum = 30)** | | | | |

*Statistical analysis. Power analysis*: Using the assumption that the distribution of total stomatitis disease activity index scores is approximately normally distributed in the population of cats studied, and that the average score in the control group is 22 (out of 30 possible) with a standard deviation of 5.37 (based on previous clinical experience), a sample size of 10 cats per group would permit detection of a decline of 40% (9 units of scoring) to be statistically significant (P<0.05) with approximately 95% power using a two-group Student's T-test.

*Data analysis*: For non-continuous ranked data (client and doctor stomatitis index scores, ranked 0-3), a Friedman test is used to compare scores between control and treatment cats (Minitab 15 statistical software, version 15.1.30.0;Minitab, State College, PA, USA). A repeated-measures ranked ANOVA followed by Bonferroni's multiple comparison test is used to compare stomatitis index scores over time (GraphPad InStat version 3.06 for Windows; GraphPad, La Jolla, CA, USA). A p-value of <0.05 is considered statistically significant for all analyses.

### Example 2

### Case Studies

To date, 10 cats have received autologous MSC treatment regimes, and one cat received intralesional treatments. All ten cats had severe chronic gingivostomatitis with the duration ranging from 1-9 years that was not responsive to full-mouth extractions, corticosteroids and antibiotic treatments. One cat was not responsive to cyclosporine treatment as well. All cats underwent general anesthesia and full mouth radiographs were obtained to rule out the presence of retaining roots or presence of other (i.e., neoplastic) oral diseases. In addition, biopsy of the affected oral mucosa was performed on all cases. A comprehensive blood work (CBC and serum biochemistry) was obtained prior to treatment, at regular interval of 1, 3 and 6 months following treatments. The cats were evaluated by 2 experienced clinicians. Clinicians and client/owners provided input for the stomatitis disease activity index.

Overall, the 10 cats aged 4 ― 14.5 years (mean 9.4 years), weighing 3.8 ― 7.2 kg (mean 5.0 kg) were included in this study. The first cat (cat 1) was initially administered 10 million cells and did not experience any clinical response. Six weeks later, a second dose of 20 million cells were administered and no appreciable response was noted 4 weeks post administration. The owner of this cat (cat #1) elected to drop out of the study and the cat was euthanized. The second cat (cat #2) received 2 administrations 1 month apart. The first administration yielded a minor clinical improvement. However, the 2nd administration yielded a profound clinical improvement with resolution of inflammation and pain 3-4 months from the beginning of the study. This cat has successfully exited the study free of gingivostomatitis and without oral pain and continues to be free of FCGS 1.5 years post treatment. The third cat (cat #3) received 2 administrations 1 month apart. The first administration yielded a favorable clinical improvement and the 2nd administration resulted in resolution of oral inflammation and pain. This cat remained free of disease for 2 months but died due to a cause unrelated to the study at the age of 15 years. The 4^{th}, 5^{th}, and 6^{th} cat (cats #4, #5 and #6) had received 2 full doses with significant clinical improvement, but not complete cure in 1 cat and 2 cats that did not respond with cure but have acceptable quality of life with receiving pain medication. Cat #7 received the first dose and responded with almost complete cure within 1 month. This cat (#7) received the second dose and is currently in observation period. Cat #8 received 2 doses and the owner elected to drop out (after second treatment) due to lack of progress. Cats #9 and #10 received 2 doses and are currently under observation.

### REFERENCES

1. Arzi B, Murphy B, Cox DP, Vapniarsky N, Kass PH, Verstraete FJ: Presence and quantification of mast cells in the gingiva of cats with tooth resorption, periodontitis and chronic stomatitis. Arch. Oral Biol. 2010; 55: 148-54
2. Harley R, Gruffydd-Jones TJ, Day MJ: Immunohistochemical characterization of oral mucosal lesions in cats with chronic gingivostomatitis. J. Comp Pathol. 2011; 144: 239-50
3. Healey KA, Dawson S, Burrow R, Cripps P, Gaskell CJ, Hart CA, Pinchbeck GL, Radford AD, Gaskell RM: Prevalence of feline chronic gingivo-stomatitis in first opinion veterinary practice. J. Feline Med. Surg. 2007; 9: 373-81
4. Hennet PR, Camy GA, McGahie DM, Albouy MV: Comparative efficacy of a recombinant feline interferon omega in refractory cases of calicivirus-positive cats with caudal stomatitis: a randomised, multi-centre, controlled, double-blind study in 39 cats. J. Feline Med. Surg. 2011; 13: 577-87
5. Lommer MJ, Verstraete FJ: Concurrent oral shedding of feline calicivirus and feline herpesvirus 1 in cats with chronic gingivostomatitis. Oral Microbiol. Immunol. 2003; 18: 131-4
6. Pedersen NC: Inflammatory oral cavity diseases of the cat. Vet. Clin. North Am. Small Anim. Pract. 1992; 22: 1323-45
7. Dowers KL, Hawley JR, Brewer MM, Morris AK, Radecki SV, Lappin MR: Association of Bartonella species, feline calicivirus, and feline herpesvirus 1 infection with gingivostomatitis in cats. J. Feline Med. Surg. 2010; 12: 314-21
8. Knowles JO, McArdle F, Dawson S, Carter SD, Gaskell CJ, Gaskell RM: Studies on the role of feline calicivirus in chronic stomatitis in cats. Vet. Microbiol. 1991; 27: 205-19
9. Sims TJ, Moncla BJ, Page RC: Serum antibody response to antigens of oral gram-negative bacteria by cats with plasma cell gingivitis-pharyngitis. J. Dent. Res. 1990; 69: 877-82
10. Hennet P: Chronic gingivostomatitis in cats: long term follow-up of 30 cases treated by dental extractions. J. Vet. Dent. 1997; 15-21
11. Borjesson DL, Peroni JF: The regenerative medicine laboratory: facilitating stem cell therapy for equine disease. Clin. Lab. Med. 2011; 31: 109-23
12. Quimby JM, Webb TL, Gibbons DS, Dow SW: Evaluation of intrarenal mesenchymal stem cell injection for treatment of chronic kidney disease in cats: a pilot study. J. Feline Med. Surg. 2011; 13: 418-26
13. Webb TL, Quimby JM, Dow SW: In vitro comparison of feline bone marrow-derived and adipose tissue-derived mesenchymal stem cells. J. Feline Med. Surg. 2012; 14: 165-8
14. Wood JA, Chung DJ, Park SA, Zwingenberger AL, Reilly CM, Ly I, Walker NJ, Vernau W, Hayashi K, Wisner ER, Cannon MS, Kass PH, Cherry SR, Borjesson DL, Russell P, Murphy CJ: Periocular and intra-articular injection of canine adipose-derived mesenchymal stem cells: an in vivo imaging and migration study. J Ocul Pharmacol Ther. 2012; 28(3):307-17
15. Dominici M, Le BK, Mueller I, Slaper-Cortenbach I, Marini F, Krause D, Deans R, Keating A, Prockop D, Horwitz E: Minimal criteria for defining multipotent mesenchymal stromal cells. The International Society for Cellular Therapy position statement. Cytotherapy 2006; 8: 315-7
16. Vieira NM, Brandalise V, Zucconi E, Secco M, Strauss BE, Zatz M: Isolation, characterization, and differentiation potential of canine adipose-derived stem cells. Cell Transplant. 2010; 19: 279-89
17. Kassem M, Kristiansen M, Abdallah BM: Mesenchymal stem cells: cell biology and potential use in therapy. Basic Clin. Pharmacol. Toxicol. 2004; 95: 209-14
18. Vidal MA, Walker NJ, Napoli E, Borjesson DL: Evaluation of senescence in mesenchymal stem cells isolated from equine bone marrow, adipose tissue, and umbilical cord tissue. Stem Cells Dev. 2012; 21: 273-83
19. Beggs KJ, Lyubimov A, Borneman JN, Bartholomew A, Moseley A, Dodds R, Archambault MP, Smith AK, McIntosh KR: Immunologic consequences of multiple, high-dose administration of allogeneic mesenchymal stem cells to baboons. Cell Transplant. 2006; 15: 711-21
20. Martinello T, Bronzini I, Maccatrozzo L, Mollo A, Sampaolesi M, Mascarello F, Decaminada M, Patruno M: Canine adipose-derived-mesenchymal stem cells do not lose stem features after a long-term cryopreservation. Res. Vet. Sci. 2011; 91: 18-24
21. Sun CK, Chang CL, Lin YC, Kao YH, Chang LT, Yen CH, Shao PL, Chen CH, Leu S, Yip HK: Systemic administration of autologous adipose-derived mesenchymal stem cells alleviates hepatic ischemia-reperfusion injury in rats. Crit. Care Med. 2012; 40: 1279-90
22. Ben-Ami E, Berrih-Aknin S, Miller A: Mesenchymal stem cells as an immunomodulatory therapeutic strategy for autoimmune diseases. Autoimmun. Rev. 2011; 10: 410-5
23. Corcione A, Benvenuto F, Ferretti E, Giunti D, Cappiello V, Cazzanti F, Risso M, Gualandi F, Mancardi GL, Pistoia V, Uccelli A: Human mesenchymal stem cells modulate B-cell functions. Blood 2006; 107: 367-72
24. Le BK, Pittenger M: Mesenchymal stem cells: progress toward promise. Cytotherapy. 2005; 7: 36-45
25. Peroni JF, Borjesson DL: Anti-inflammatory and immunomodulatory activities of stem cells. Vet. Clin. North Am. Equine Pract. 2011; 27: 351-62
26. Singer NG, Caplan AI: Mesenchymal stem cells: mechanisms of inflammation. Annu. Rev. Pathol. 2011; 6: 457-78
27. Prasad VK, Lucas KG, Kleiner GI, Talano JA, Jacobsohn D, Broadwater G, Monroy R, Kurtzberg J: Efficacy and safety of ex vivo cultured adult human mesenchymal stem cells (Prochymal) in pediatric patients with severe refractory acute graft-versus-host disease in a compassionate use study. Biol. Blood Marrow Transplant. 2011; 17: 534-41
28. Zhang LS, Liu QF, Huang K, Zhang Y, Fan ZP, Huang SL: [Mesenchymal stem cells for treatment of steroid-resistant chronic graft-versus-host disease]. Zhonghua Nei Ke. Za Zhi. 2009; 48: 542-6
29. Carrade DD, Lame MW, Kent MS, Clark KC, Walker NJ, and Borjesson DL. Comparative analysis of the immunomodulatory properties of equine adult-derived mesenchymal stem cells. Cell Med. 2012; 4(1):1-11
30. Lee PH, Kim JW, Bang OY, Ahn YH, Joo IS, Huh K: Autologous mesenchymal stem cell therapy delays the progression of neurological deficits in patients with multiple system atrophy. Clin. Pharmacol. Ther. 2008; 83: 723-30
31. Locke M, Windsor J, Dunbar PR: Human adipose-derived stem cells: isolation, characterization and applications in surgery. ANZ. J. Surg. 2009; 79: 235-44
32. Zhao W, Li JJ, Cao DY, Li X, Zhang LY, He Y, Yue SQ, Wang DS, Dou KF: Intravenous injection of mesenchymal stem cells is effective in treating liver fibrosis. World J. Gastroenterol. 2012; 18: 1048-58
33. Carrade DD, Owens SD, Galuppo LD, Vidal MA, Ferraro GL, Librach F, Buerchler S, Friedman MS, Walker NJ, Borjesson DL: Clinicopathologic findings following intra-articular injection of autologous and allogeneic placentally derived equine mesenchymal stem cells in horses. Cytotherapy. 2011; 13: 419-30
34. Kolf CM, Cho E, Tuan RS: Mesenchymal stromal cells. Biology of adult mesenchymal stem cells: regulation of niche, self-renewal and differentiation. Arthritis Res. Ther. 2007; 9: 204
35. Carrade DD, Affolter VK, Outerbridge CA, Watson JL, Galuppo LD, Buerchler S, Kumar V, Walker NJ, Borjesson DL: Intradermal injections of equine allogeneic umbilical cord-derived mesenchymal stem cells are well tolerated and do not elicit immediate or delayed hypersensitivity reactions. Cytotherapy. 2011; 13: 1180-92

## Claims

1. An effective amount of adipose-derived stem cells (AdMSCs) for the use in a method of preventing, reducing, mitigating, ameliorating and/or reversing stomatitis in a subject mammal in need thereof.

2. The effective amount of AdMSCs for the use of claim 1, wherein the AdMSCs are autologous, syngeneic, allogenic or xenogeneic to the subject mammal.

3. The effective amount of AdMSCs for the use of claims 1 to 2, wherein the subject mammal is a feline.

4. The effective amount of AdMSCs for the use of any one of claims 1 to 3, wherein:
a) the subject mammal is exhibiting symptoms of stomatitis; and/or
b) the subject mammal was not responsive to corticosteroids and/or antibiotic treatments.

5. The effective amount of AdMSCs for the use of claim 4, wherein the subject mammal was not responsive to full-mouth extractions or near full-mouth extractions.

6. The effective amount of AdMSCs for the use of any one of claims 1 to 5, wherein the AdMSCs:
a) the stomatitis is chronic; and/or
b) the stomatitis is gingivostomatitis.

7. The effective amount of AdMSCs for the use of any one of claims 1 to 6, wherein the AdMSCs:
a) are autologous to the subject mammal;
b) are syngeneic to the subject mammal; or
c) are allogeneic to the subject mammal.

8. The effective amount of AdMSCs for the use of any one of claims 1 to 7, wherein the AdMSCs are CD44⁺, CD90⁺ and CD105⁺, and CD34⁻, CD45⁻ and MHC class II⁻.

9. The effective amount of AdMSCs for the use of any one of claims 1 to 8, wherein the AdMSCs;
a) are fresh;
b) have been cultured in vitro for at least 1 passage;
c) are a population of cells that is at least about 90 % viable; and/or
d) have not been frozen.

10. The effective amount of AdMSCs for the use of any one of claims 1 to 9, wherein the AdMSCs;
a) have been cultured in serum-free cell culture media;
b) have been cultured in cell culture media comprising serum proteins allogeneic to the subject mammal;
c) the AdMSCs are substantially free of serum proteins xenogeneic to the subject mammal; and/or
d) are substantially free of bovine serum proteins.

11. The effective amount of AdMSCs for the use of any one of claims 1 to 10, wherein the AdMSCs;
a) are administered at a rate of about 1 million to about 10 million cells per minute;
b) are administered systemically;
c) are administered intravenously; and/or
d) are administered in multiple administrations.

12. The effective amount of AdMSCs for the use of any one of claims 1 to 6, 8, or 10-11, wherein the AdMSCs are allogeneic to the subject mammal, have been cultured *in vitro* for 2 to 8 passages, are not frozen prior to administration, and comprise a population of cells that is at least about 90% viable.

13. The effective amount of AdMSCs for the use of any one of claims 1 to 12, wherein:
a) at least about 1 million AdMSCs/kg subject are administered;
b) about 1 million to about 10 million AdMSCs/kg subject are administered;
c) at least about 5 million AdMSCs are administered; and/or
d) about 5 million to about 100 million AdMSCs are administered.

## Patentansprüche

1. Wirksame Menge von aus Fettgewebe stammenden Stammzellen (AdMSCs) zur Verwendung in einem Verfahren zum Verhüten, Reduziern, Mildern, Verbessern und/oder Umkehren von Stomatitis bei einem dies benötigenden betreffenden Säugetier.

2. Wirksame Menge von AdMSCs zur Verwendung nach Anspruch 1, wobei die AdMSCs autolog, syngen, allogen oder xenogen zu dem betreffenden Säugetier sind.

3. Wirksame Menge von AdMSCs zur Verwendung nach den Ansprüchen 1 bis 2, wobei das betreffende Säugetier eine Katze ist.

4. Wirksame Menge von AdMSCs zur Verwendung nach einem der Ansprüche 1 bis 3, wobei:
a) das betreffende Säugetier Stomatitis-Symptome aufweist; und/oder
b) das betreffende Säugetier nicht auf Corticosteroide und/oder Antibiotikabehandlungen angesprochen hat.

5. Wirksame Menge von AdMSCs zur Verwendung nach Anspruch 4, wobei das betreffende Säugetier nicht auf eine Komplettextraktion oder eine nahezu Komplettextraktion angesprochen hat.

6. Wirksame Menge von AdMSCs zur Verwendung nach einem der Ansprüche 1 bis 5, wobei die AdMSCs [sic]:
a) die Stomatitis chronisch ist; und/oder
b) die Stomatitis eine Gingivostomatitis ist.

7. Wirksame Menge von AdMSCs zur Verwendung nach einem der Ansprüche 1 bis 6, wobei die AdMSCs:
a) autolog zu dem betreffenden Säugetier sind;
b) syngen zu dem betreffenden Säugetier sind; oder
c) allogen zu dem betreffenden Säugetier sind.

8. Wirksame Menge von AdMSCs zur Verwendung nach einem der Ansprüche 1 bis 7, wobei die AdMSCs CD44⁺,CD90⁺ und CD105⁺ und CD34⁻, CD45⁻ und MHC-Klasse II⁻ sind.

9. Wirksame Menge von AdMSCs zur Verwendung nach einem der Ansprüche 1 bis 8, wobei die AdMSCs:
a) frisch sind;
b) über wenigstens eine Passage in vitro kultiviert wurden;
c) eine Zellpopulation sind, die zu wenigstens etwa 90 % lebensfähig ist; und/oder
d) nicht eingefroren wurden.

10. Wirksame Menge von AdMSCs zur Verwendung nach einem der Ansprüche 1 bis 9, wobei die AdMSCs:
a) in serumfreien Zellkulturmedien kultiviert wurden;
b) in Zellkulturmedien kultiviert wurden, die Serumproteine umfassen, die allogen zu dem betreffenden Säugetier sind;
c) im Wesentlichen frei von Serumproteinen sind, die xenogen zu dem betreffenden Säugetier sind; und/oder
d) im Wesentlichen frei von Rinderserumproteinen sind.

11. Wirksame Menge von AdMSCs zur Verwendung nach einem der Ansprüche 1 bis 10, wobei die AdMSCs:
a) mit einer Rate von etwa 1 Million bis etwa 10 Millionen Zellen pro Minute verabreicht werden;
b) systemisch verabreicht werden;
c) intravenös verabreicht werden; und/oder
d) in mehreren Verabreichungen verabreicht werden.

12. Wirksame Menge von AdMSCs zur Verwendung nach einem der Ansprüche 1 bis 6, 8 oder 10 bis 11, wobei die AdMSCs allogen zu dem betreffenden Säugetier sind, über 2 bis 8 Passagen *in vitro* kultiviert wurden, vor der Verabreichung nicht eingefroren werden und eine Zellpopulation umfassen, die zu wenigstens etwa 90 % lebensfähig ist.

13. Wirksame Menge von AdMSCs zur Verwendung nach einem der Ansprüche 1 bis 12, wobei:
a) wenigstens etwa 1 Million AdMSCs/kg des Subjekts verabreicht werden;
b) etwa 1 Million bis etwa 10 Millionen AdMSCs/kg des Subjekts verabreicht werden;
c) wenigstens etwa 5 Millionen AdMSCs verabreicht werden; und/oder
d) etwa 5 Millionen bis etwa 100 Millionen AdMSCs verabreicht werden.

## Revendications

1. Une dose efficace de cellules souches dérivées de tissus adipeux (AdMSC) pour utilisation dans un procédé destiné à prévenir, réduire, soulager, améliorer et/ou enrayer une stomatite chez un sujet mammifère le nécessitant.

2. La dose efficace d'AdMSC pour utilisation de la revendication 1, les AdMSC étant autologues, syngéniques, allogéniques ou xénogéniques au sujet mammifère.

3. La dose efficace d'AdMSC pour utilisation des revendications 1 ou 2, le sujet mammifère étant un félin.

4. La dose efficace d'AdMSC pour utilisation d'une quelconque des revendications 1 à 3 :
a) le sujet mammifère présentant des symptômes de stomatite ; et/ou
b) le sujet mammifère n'ayant pas été sensible à des traitements aux corticostéroïdes et/ou antibiotiques.

5. La dose efficace d'AdMSC pour utilisation de la revendication 4, le sujet mammifère n'ayant pas été sensible à des extractions de la totalité ou presque totalité des dents.

6. La dose efficace d'AdMSC pour utilisation d'une quelconque des revendications 1 à 5 :
a) la stomatite étant chronique ; et/ou
b) la stomatite étant une gingivostomatite.

7. La dose efficace d'AdMSC pour utilisation d'une quelconque des revendications 1 à 6, dans laquelle les AdMSC :
a) sont autologues au sujet mammifère ;
b) sont syngéniques au sujet mammifère ; ou
c) sont allogéniques au sujet mammifère.

8. La dose efficace d'AdMSC pour utilisation d'une quelconque des revendications 1 à 7, dans laquelle les AdMSC sont CD44⁺, CD90⁺ et CD105⁺, et CD34⁻, CD45⁻ et MHC classe II⁻.

9. La dose efficace d'AdMSC pour utilisation d'une quelconque des revendications 1 à 8, dans laquelle les AdMSC :
a) sont fraîches ;
b) ont été cultivées in vitro pendant au moins 1 passage ;
c) sont une population de cellules qui est viable à au moins 90 % ; et/ou
d) n'ont pas été congelées.

10. La dose efficace d'AdMSC pour utilisation d'une quelconque des revendications 1 à 9, dans laquelle les AdMSC :
a) ont été cultivées dans des milieux de culture cellulaire sans sérum ;
b) ont été cultivées dans des milieux de culture cellulaire comprenant des protéines sériques allogéniques au sujet mammifère ;
c) sont sensiblement dépourvues de protéines sériques xénogéniques au sujet mammifère ; et/ou
d) sont sensiblement dépourvues de protéines sériques bovines.

11. La dose efficace d'AdMSC pour utilisation d'une quelconque des revendications 1 à 10, dans laquelle les AdMSC :
a) sont administrées à un taux d'environ 1 million à environ 10 million de cellules par minute ;
b) sont administrées par voie systémique ;
c) sont administrées par voie intraveineuse ; et/ou
d) sont administrées en de multiples administrations.

12. La dose efficace d'AdMSC pour utilisation d'une quelconque des revendications 1 à 6, 8, ou 10-11, dans laquelle les AdMSC sont allogéniques au sujet mammifère, ont été cultivées in vitro pendant 2 à 8 passages, n'ont pas été congelées avant l'administration, et comprennent une population de cellules qui est viable à au moins 90%.

13. La dose efficace d'AdMSC pour utilisation d'une quelconque des revendications 1 à 12, dans laquelle :
a) au moins environ 1 million d'AdMSC/kg du sujet sont administrées ;
b) environ 1 million à environ 10 millions d'AdMSC/kg du sujet sont administrées ;
c) au moins environ 5 millions d'AdMSC sont administrées ; et/ou
d) environ 5 millions à environ 100 millions d'AdMSC sont administrées.
